(19) Europäisches Patentamt
European Patent Office
Office européen des brevets

(11) Publication number: **0 249 194 A2**

# (12) EUROPEAN PATENT APPLICATION

(21) Application number: 87108314.3

(22) Date of filing: 09.06.87

(51) Int. Cl.[4]: A61K 31/557 , A61K 47/00 , A61K 7/06

(30) Priority: 09.06.86 US 870391

(43) Date of publication of application: 16.12.87 Bulletin 87/51

(84) Designated Contracting States: AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: Ritter, Lawrence
25 Hillcrest Road
Suffern New York 10901(US)
Inventor: Lawter, James Ronald
35 Glen Drive
Goshen New York 10924(US)
Inventor: Sutherland, George Leslie
42 Sky Meadow Road
Suffern New York 10901(US)

(74) Representative: Wächtershäuser, Günter, Dr.
Tal 29
D-8000 München 2(DE)

## (54) Topical prostaglandin formulations.

(57) An E-type prostaglandin, together with pharmaceutically acceptable carrier for such, useful in treatment of androgenetic alopecia are described.

EP 0 249 194 A2

# TOPICAL PROSTAGLANDIN FORMULATIONS

## BACKGROUND OF THE INVENTION

Prostaglandins are a group of cyclic fatty acids that possess diverse and potent biologic activities affecting cellular function in every organ system. The parent compound, prostanoic acid, contains a 20 carbon chain with a cyclopentane ring.

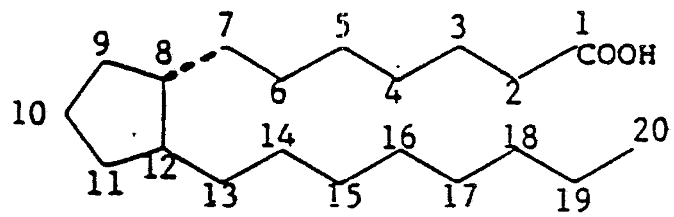

PROSTANOIC ACID

Variations in the number and position of the double bonds and hydroxyl groups determine the physiologic activities of the various prostaglandins.

Conventionally, prostaglandins are divided into types E, F, A, B, C and D based on functions in the cyclopentane ring. Numerical subscripts refer to the number of unsaturations in the side chains and, $\alpha$ or $\beta$ subscripts refer to the configuration of the substituents in the ring. The naturally occurring prostaglandins are types E, F, A and B. All naturally ocurring prostaglandins have a trans 13,14 position bond and an hydroxyl group at $C_{15}$.

The E-and F-type prostaglandins possess an additional hydroxyl at $C_{11}$. At $C_9$, E-type prostaglandins have a carbonyl function while F-type prostaglandins have an hydroxyl. In general, A-and B-type prostaglandins may be regarded as dehydration products of E-type prostaglandins; i.e., the removal of the $C_{11}$ hydroxyl and the formation of a double bond in the cyclopentane ring.

The known biologic activities of prostaglandins of the E-type include activities as hypotensive agents, bronchodilators, and gastric acid secretion inhibition agents. [Bergstrom, et al., PHARMACOL., Rev., 20:1 (1968)]. However, pharmaceutical use of E-type prostaglandins has been impeded by their instability. E-type prostaglandins generally decompose slowly at room temperature and above, which decomposition is accelerated in the presence of small amounts of acid or base. Accordingly, E-type prostaglandins are unstable in pharmaceutical formulations containing water or hydroxylic compounds. Even in neutral, aqueous solution or in neat state there is a gradual decomposition of E-type prostaglandins to A-and B-type prostaglandins.

Good stability of the E-type prostaglandins has been observed in some solutions and in pure form at temperatures of -20°C or lower. However, storage at such temperatures is impractical. Some success at stabilization at room temperature has been reported when non-alcoholic compounds such ethyl acetate and chloroform are employed as solvents for E-type prostaglandins. Such solvents, however, are unsuitable for pharmaceutical dosage applications.

More recently, good stability of E-type prostaglandins was reported with use of triethyl citrate as a solvent (U. S. Patent No. 4,211,793) and with use of hydroxylated derivatives of fatty acids (U. S. Patent No. 4,431,833).

Although hair serves no vital function in humans, the psychological impact from excess hair growth or loss of hair can be enormous. For some men in particular, as a result of aging and predisposing genetic factors, scalp baldness occurs in stages often resulting in a complete loss of scalp hair. This baldness is known as male pattern baldness or androgenetic alopecia. It is believed that an accumulation of 5-$\alpha$-dihydro testosterone, a tisue-active androgen, in some scalp hair follicles over time causes the regression of hair growth in such follicles. However, androgen production is no higher in men with androgenetic alopecia than in those with full scalps of hair.

There is no effective treatment for androgenetic alopecia at the present time. There have been some reports that minoxidil, a potent vasodilator, has been effective in causing scalp hair growth in patients with androgenetic alopecia. However, contrary to some popular belief, there is no evidence of a vascular deficit to the scalp in patients with androgenetic alopecia.

THE INVENTION

The E-type prostaglandin with which this invention is most particularly concerned has the following structure:

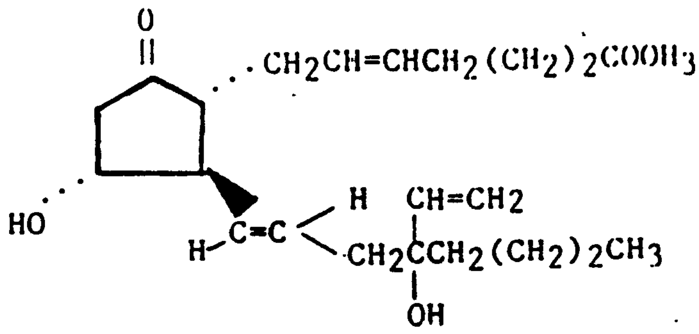

Methyl (±)-[11α,5Z(5E), 13E, 16R and 16S]-16 ethenyl-11,16-dihydroxy-9-oxoprosta-5,13-dien-1-oate (Chemical Abstracts nomenclature)

This comound and uses thereof are described in U. S. Patent Nos. 4,198,521 and 4,311,707, both incorporated herein by reference. Such prostaglandin is a vasodilator, and can be applied topically for such effect. The prostaglandin is known by the name viprostol.

It has been discovered that viprostol topically applied to the scalp can promote hair growth in males with male pattern baldness. The mechanism by which viprostol is effective in promoting such growth is unknown. Additionally, a vehicle for topical application of viprostol to the scalp of an individual with androgenetic alopecia has been developed. The vehicle is non-greasy and impedes decomposition of viprostol at room temperature.

As more fully discussed hereinabove, a principal problem encountered with pharmacological utilization of prostaglandins resides in their relatively unstable nature in pharmaceutical formulations.The few sucessful attempts at providing stable formulations have resulted in oily, liquid compositions, such as the triethyl citrate formulation of U.S. 4,211,793. Such formulation is unsatisfactory for topical application as it results in greasy build-up on the skin. The invention, accordingly, includes a vehicle in which viprostol is stable at room temperature, and which also has good isometric properties. The vehicle does not leave a perceptible, greasy film on the skin to which it is applied. It also has sufficient viscosity to avoid rapid run-off from the site of application.

In its broadest sense the topical composition containing viprostol may be described by the following formula:

| Ingredient | Concentration (% by weight) |
|---|---|
| Viprostol | 0.001 to 2.0 |
| Polydimethylsiloxane (20 to 1,000,000 centistokes) | 1 to 50 |
| Cosolvent* | 2 to 60 |
| Vehicle** ... qs to ... | 100 |

* The cosolvent may be selected from esters such as $C_{12}$-$C_{15}$ alcohols benzoate, isopropyl palmitate, isopropyl myristate, diisopropyl adipate, octyl hydroxystearate, PPG-2 myristyl ether propionate, almond oil, etc.

**The vehicle may be selected from volatile silicones such as octamethylcyclotetrasiloxane, decamethylcyclopentasiloxane, hexamethyldisiloxane and other low molecular weight polydimethylsiloxanes such as Dow Corning 200 Fluid 1.0 centistokes and 1.5 centistokes and pharmaceutically acceptable chlorofluorocarbons (such as the Freons).

In a specific scense, the topical composition according to the present invention may be described by the following formula:

## Formulation A

| Ingredient | Concentration (% by weight) |
|---|---|
| Viprostol | 0.04 |
| Polydimethylsiloxane (12,500 centistokes) | 15 |
| $C_{12}$-$C_{15}$ Alcohols Benzoate | 20 |
| Cyclomethicone (Octamethylcyclotetrasiloxane ... qs to ... | 100 |

The following are studies which compare the stability of viprostol as a bulk liquid (neat) stored in glass ampules, as an ethanol solution stored in glass and as part of the above topical formulation stored in glass ampules.

## Stability of Viprostol Compositions
## (% of Initial Potency)

| Storage Conditions | Neat Compound | Ethanol Solution | Formulation A |
|---|---|---|---|
| Initial | 100 | 100 | 100 |
| 23°C 1 month | - | - | 97 |
| 2 months | 99 | - | - |
| 3 months | - | 73 | 99 |
| 6 months | 87 | 50 | - |
| 12 months | 20 | - | 99 |
| 18 months | 12 | - | - |
| 37°C 2 weeks | - | - | 101 |
| 1 month | - | 38 | 97 |
| 3 months | - | - | 100 |
| 37°C 75% relative humidity 2 weeks | - | - | 100 |
| 75% relative humidity 1 month | - | - | 94 |
| 42°C 2 weeks | - | - | 100 |
| 1 month | - | 37 | - |
| 2 months | 83 | 20 | - |
| 56°C 2 weeks | - | 15 | 99 |
| 1 month | 32 | 0 | - |
| 2 months | 0 | 0 | - |

The following description details the preparation of a typical formulation encompassed by this invention.

**Formulation A - Topical Solution Providing 120 mcg of Prostaglandin Per 0.3 ml**

| Ingredient | Concentration (% by Weight) | Quantity (mg/0.3 ml) |
|---|---|---|
| Viprostol | 0.042 | 0.12 |
| Polydimethylsiloxane (12,500 centistokes) | 15 | 43 |
| $C_{12}$-$C_{15}$ Alcohols Benzoate | 20 | 57.4 |
| Cyclomethicone (Octamethylcyclotetrasiloxane | qs | - |

The polydimethylsiloxane was placed in a suitable container. One half of the total quantity of cyclomethicone was added to the container and the contents were stirred until homogeneous.

Viprostol was weighed into a suitable container. Approximately 25% of the $C_{12}$-$C_{15}$ alcohols benzoate was added to this container, the mixture was stirred and that portion of the prostaglandin which dissolved was drained into a third container. The second container was rinsed three more times with the remaining $C_{12}$-$C_{15}$ alcohols benzoate. Each rinse was added to the third container.

The resulting prostaglandin solution was added to the polydimethylsiloxane-cyclomethicone solution.

The solution was agitated until homogeneous, the remaining portion of cyclomethicone was added and this final solution agitated until homogeneous and then filled into suitable containers.

Clinical studies using healthy, male volunteers (age 18-65) with vertex baldness and some recession of front hairline were performed using viprostol in Formulation A, above-described. The volunteers were not hypertensive. A bald or balding patch of scalp in the crown or vertex region near the margin of existing hairline, approximately one inch in diameter, was observed for hair growth. The hairs on such location were counted at the beginning of the study, and recounts were done periodically during the course of the study. All hair, including unpigmented vellus and pigmented intermediate and terminal hair was, counted, except as indicated hereinbelow. The amount of viprostol applied was approximately 120 mcg, which amount was applied once daily to the bald and balding areas of the scalp. The viprostol was contained in the above-described Formulation A. Approximately 0.3 ml of such formulation was topically to the scalp with an index finger and rubbing action. The scalp was clean (recently shampooed) and dry at application. The chart below summarizes the results after three months of study.

| | | Intermediate and Terminal Hair | Total Hair Count |
|---|---|---|---|
| Percent Increase in Hair from Initial Hair Count at Beginning of Study | > 10% | 75% | 75% |
| | > 20% | 68% | 64% |
| | > 30% | 63% | 56% |
| | > 50% | 70% | 39% |
| | > 70% | 33% | 29% |
| | >100% | 32% | 17% |
| | >150% | 17% | 5% |
| | >200% | 10% | 5% |
| | >300% | 3% | 0% |

Additionally, as the study proceeded beyond three months, the results wre charted as mean hair counts over time. Fig. 1 shows the total mean hair count over a six-month period of time. Fig. 2 shows the mean hair counts of intermediate and terminal hair over a six-month period of time. Fig. 3 shows the mean hair count of intermediate hair over a six-month period of time. Fig. 4 shows the mean hair count of terminal hair over a six-month period of time. Fig. 5 shows the mean hair count of vellus hair over a six-month period of time.

As seen in Figs. 1-5 and the chart provided hereinabove, topical application of small amounts of viprostol caused hair growth.

## Claims

1. A method for treating androgenetic alopecia in humans which method comprises topically applying to the affected scalp areas an effective amount of methyl (±)-[11 α, 5Z(5E), 13E, 16R and 16S]-16 ethenyl-11,16-dihydroxy-9-oxoprosta-5,13-dien-1-oate.

2. A topical, pharmaceutical composition of matter comprising from 0.0035 to 1.74 percent by weight of methyl (±)-[11 α, 5Z(5E), 13E, 16R and 16S]-16 ethenyl-11,16-dihydroxy-9-oxoprosta-5,13-dien-1-oate; from 1 to 50 percent by weight of a polydimethylsiloxane having a viscosity of from 20 to 1,000,000 centistokes; from 2 to 60 percent by weight of an ester cosolvent; and sufficient volatile solvent to provide 100% by weight.

3. The topical, pharmaceutical composition of matter according to Claim 2, wherein the prostaglandin constitutes 0.042 percent by weight of the formulation.

4. The topical, pharmaceutical composition of matter according to Claim 2, wherein the polydimethylsiloxane constitutes 15 percent by weight of the formulation and has a viscosity of 12,500 centistokes.

5. The topical, pharmaceutical composition of matter according to Claim 2, wherein the cosolvent is a $C_{12}$-$C_{15}$ alcohols benzoate, isopropyl palmitate, isopropyl myristate, diisopropyl adipate, octyl hydroxystearate, PPG-2 myristyl ether propionate, almond oil or mixtures thereof.

6. The topical, pharmaceutical composition of matter according to Claim 5 wherein the cosolvent is a $C_{12}$-$C_{15}$ alcohols benzoate and constitutes 20 percent by weight of the formulation.

7. The topical, pharmaceutical composition of matter according to claim 2 wherein the volatile solvent is a volatile silicone, such as octamethylcyclotetrasiloxane, decamethylcyclopentasiloxane, hexamethyldisiloxane, or other low molecular weight polydimethylsiloxanes, or pharmaceutically acceptable chlorofluorocarbons.

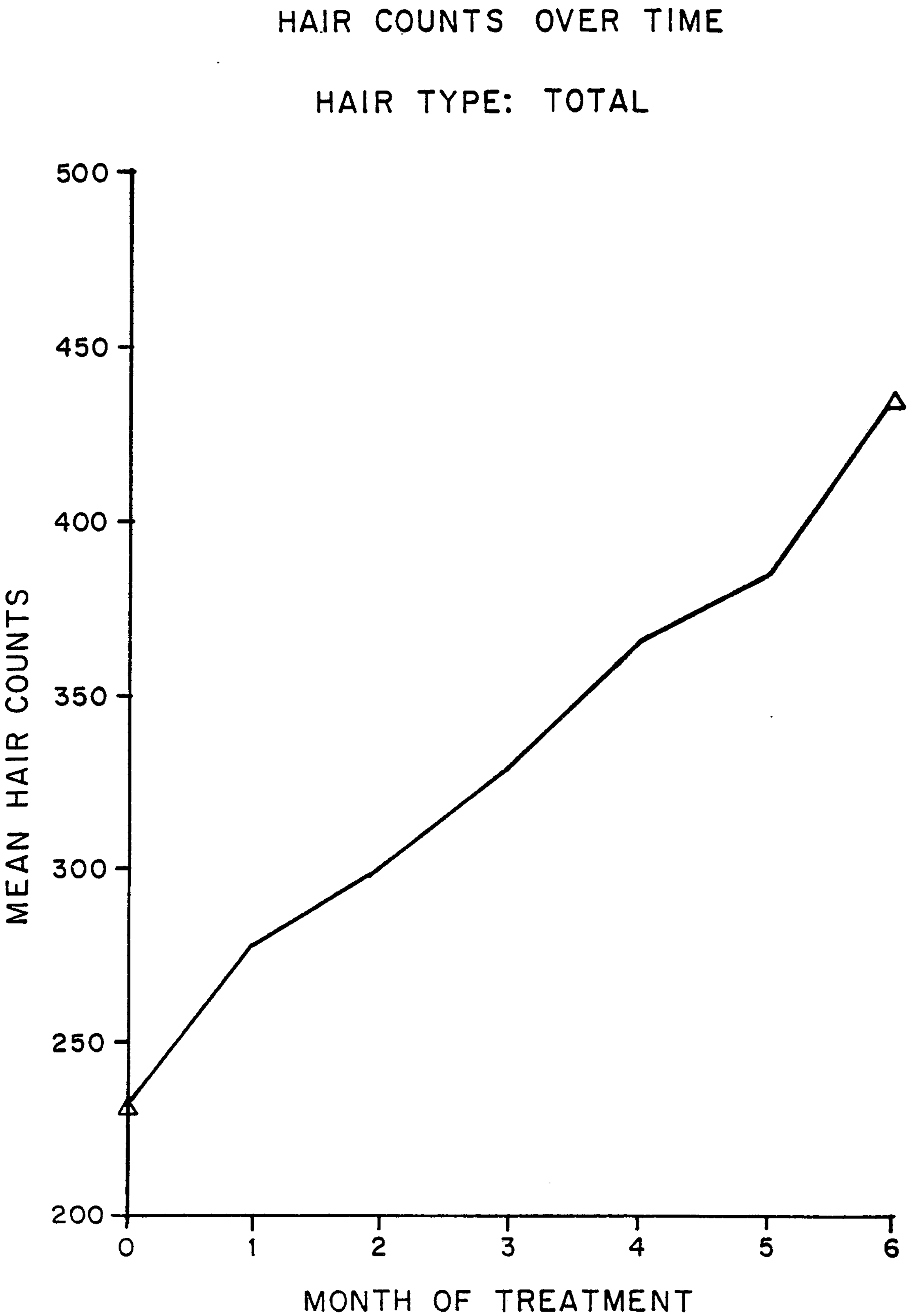
HAIR COUNTS OVER TIME
HAIR TYPE: TOTAL
MEAN HAIR COUNTS
500
450
400
350
300
250
200
0
1
2
3
4
5
6
MONTH OF TREATMENT

FIG. 1

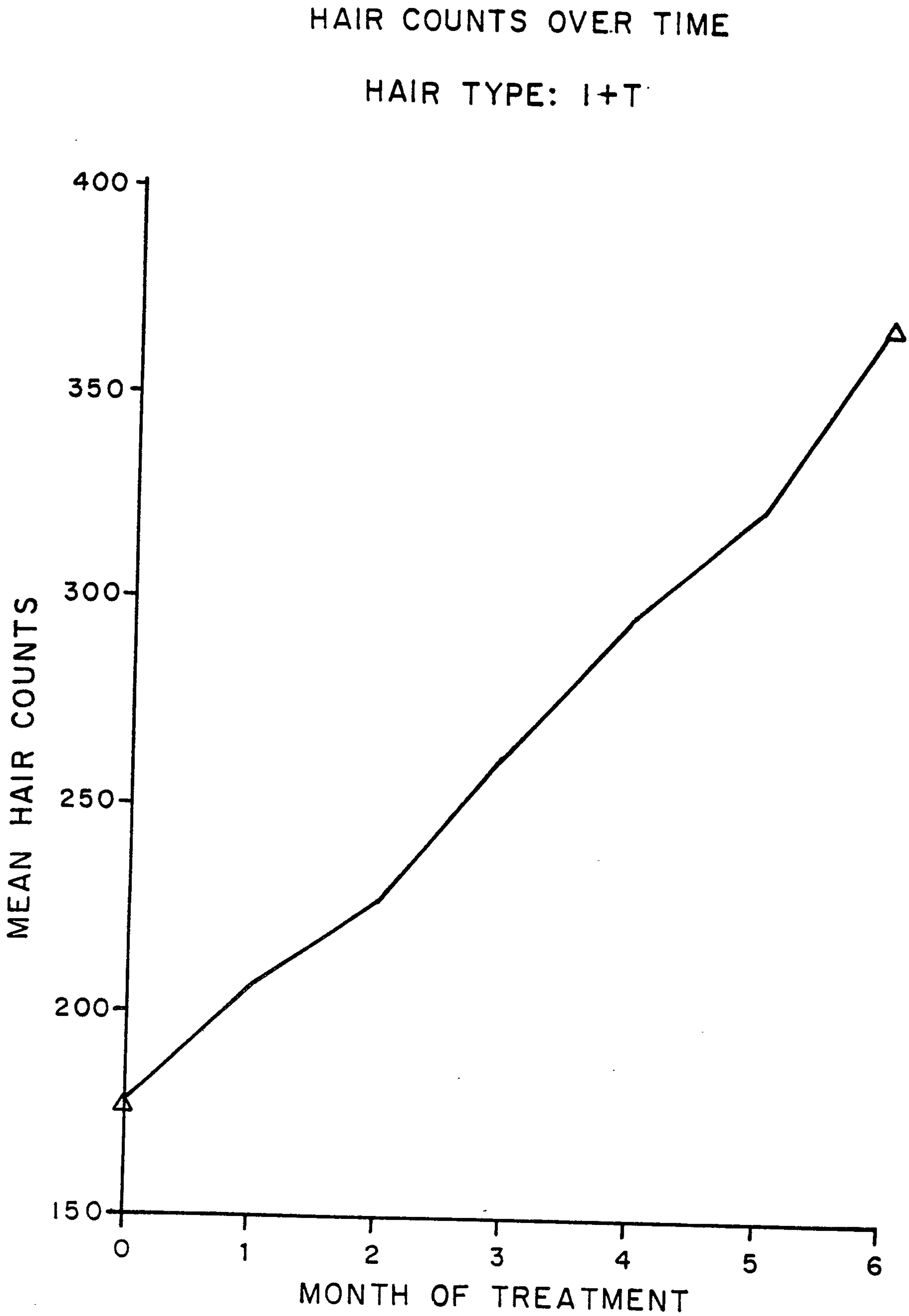
HAIR COUNTS OVER TIME
HAIR TYPE: I+T
MEAN HAIR COUNTS
400
350
300
250
200
150
0
1
2
3
4
5
6
MONTH OF TREATMENT

FIG. 2

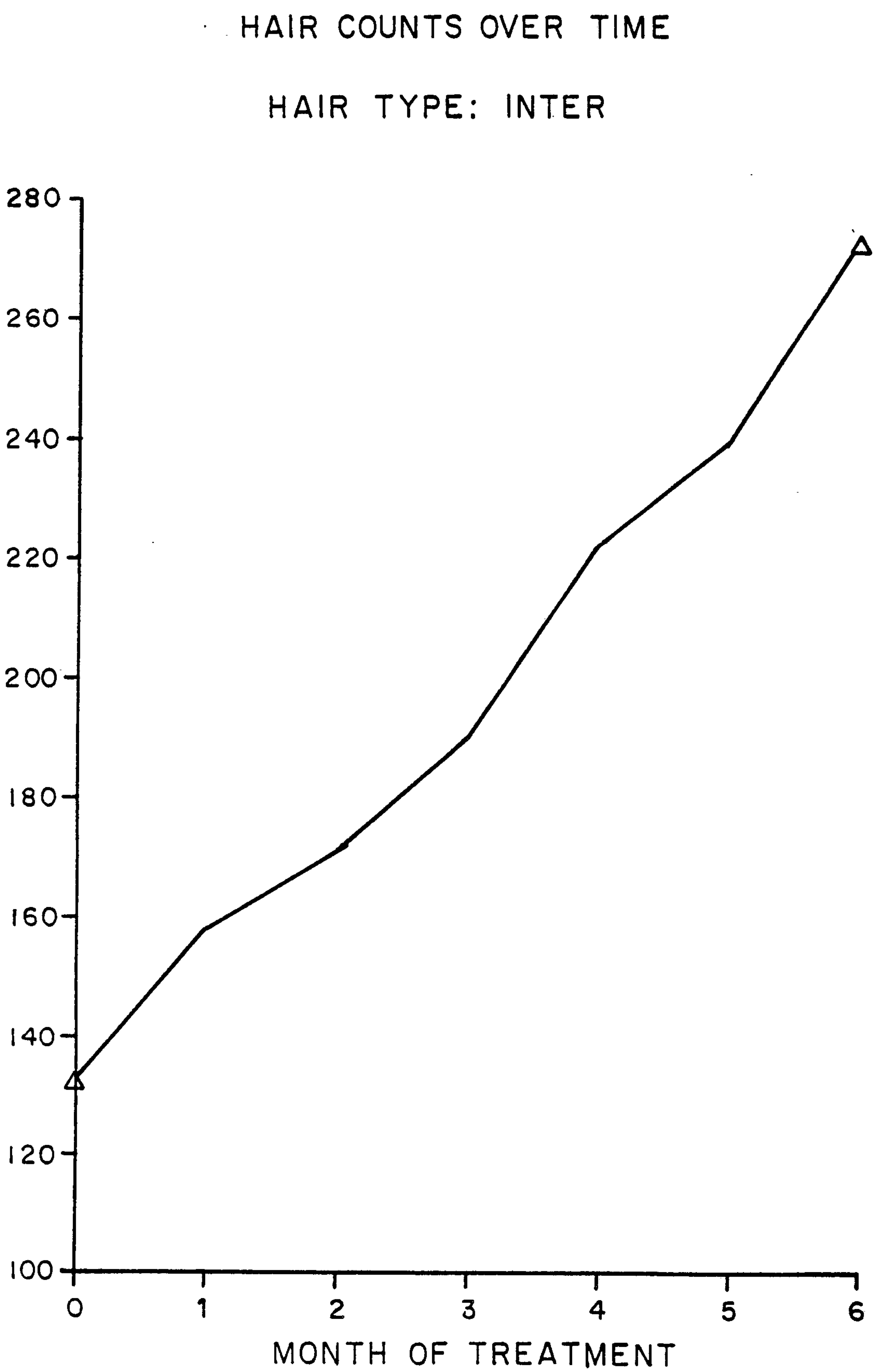
HAIR COUNTS OVER TIME
HAIR TYPE: INTER
280
260
240
220
200
180
160
140
120
100
0
1
2
3
4
5
6
MONTH OF TREATMENT

FIG. 3

HAIR COUNTS OVER TIME

HAIR TYPE: TERM

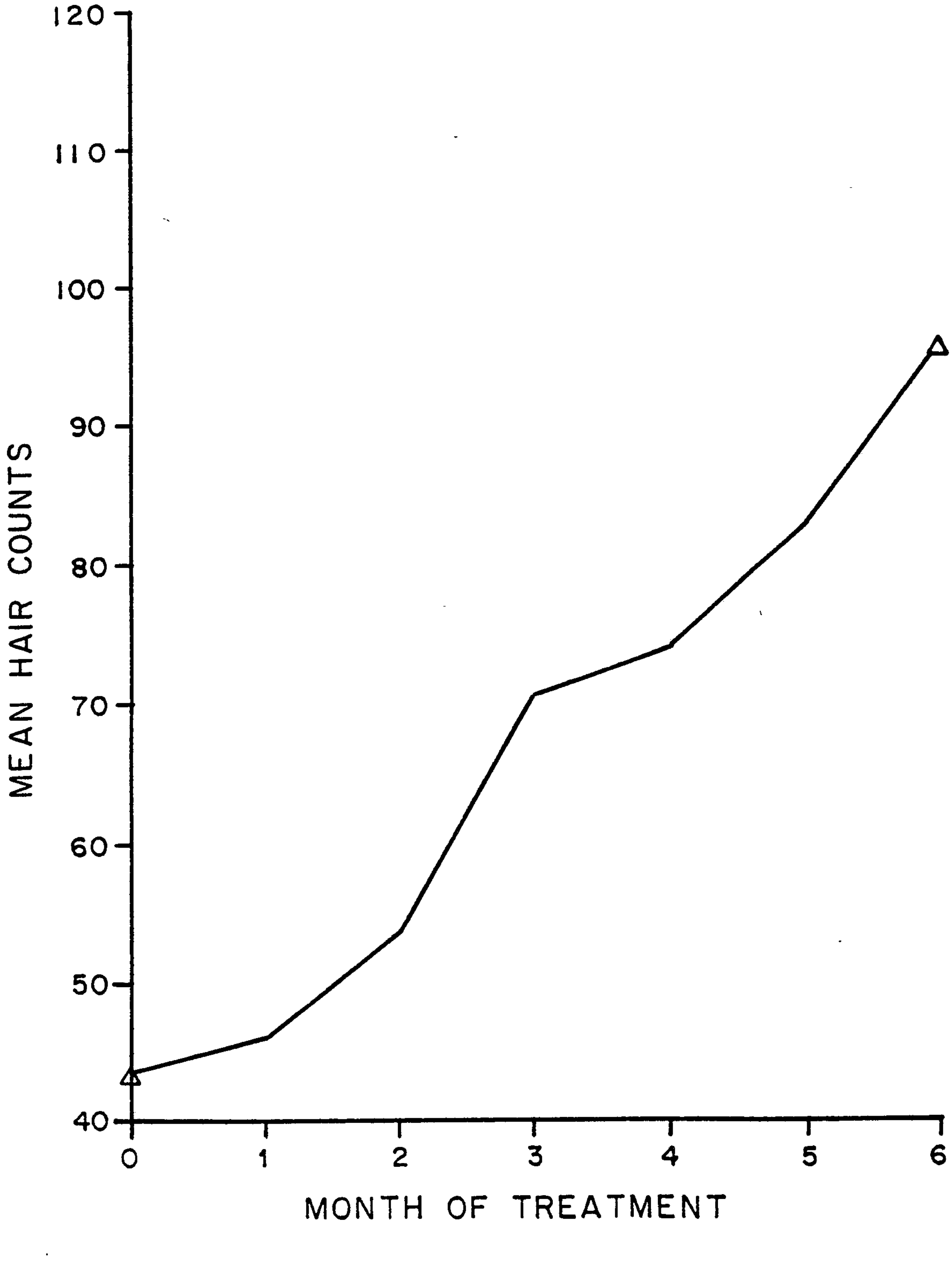


FIG. 4

HAIR COUNTS OVER TIME

HAIR TYPE: VELLUS

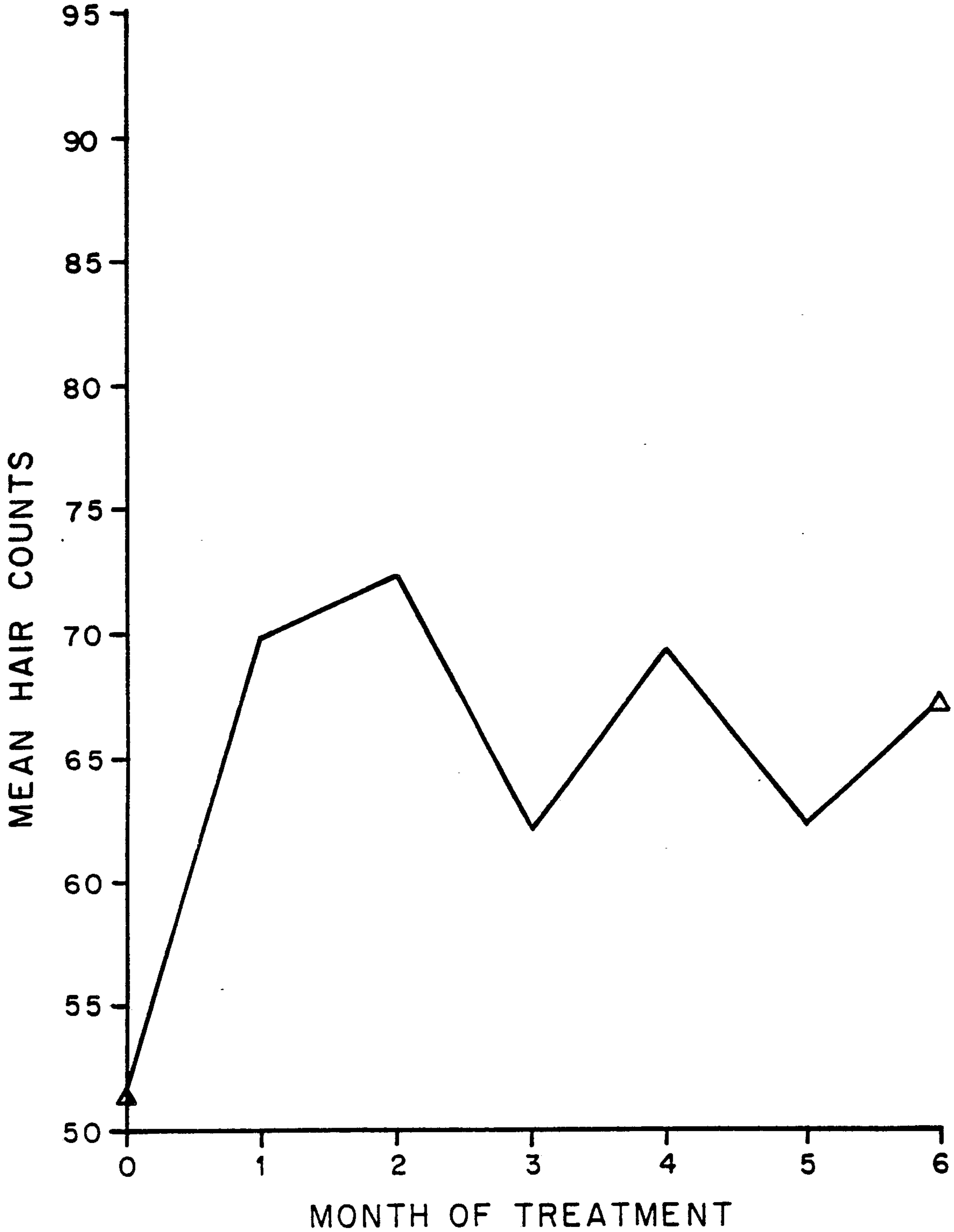


FIG. 5